# EUROPÄISCHE PATENTSCHRIFT

(11) **EP 0 913 153 B1**
(45) Veröffentlichungstag und Bekanntmachung des Hinweises auf die Patenterteilung: **26.10.2005**
(21) Anmeldenummer: 98120715.2
(22) Anmeldetag: 31.10.1998
(51) Int. Cl.: A61K 31/51, A61P 13/12, A61P 27/02

(54) **Verwendung von Thiaminen zur Herstellung eines Arzneimittels zur Prophylaxe und zur Therapie der diabetischen Retinopathie und der diabetischen Nephropathie**
Use of thiamines for the manufacture of a medicament to prevent and treat diabetic retinopathy and diabetic nephropathy
Utilisation de thiamines pour fabriquer un médicament destiné à la prévention et au traitement des rétinopathies et néphropaties dues au diabète

(30) Priorität: 03.11.1997 DE 19748417
(43) Veröffentlichungstag der Anmeldung: 06.05.1999
(73) Patentinhaber: WÖRWAG PHARMA GmbH & Co. KG, 71034 Böblingen (DE)
(72) Erfinder: Wörwag, Fritz, Dr., 70192 Stuttgart (DE)
(74) Vertreter: Mütschele, Thomas

(56) Entgegenhaltungen:
- MARGOLIS ET AL: "cocarboxylase in complex treatment of diabetic retinopathy" PROBLEMY ENDOKRINOLOGII, Bd. 15, Nr. 5, 1969, Seiten 121-122, XP002093652
- DATABASE MEDLINE1975 KASHINTSEVA: "immediate and remote results of treatment of diabetic retinopathy" XP002093653 & KASHINTSEVA: OFTALMOL ZH, Bd. 30, Nr. 6, 1975, Seiten 403-405,
- DATABASE MEDLINE1974 ROGOZINA: "our experience with complex drug therapy of diabetic retinopathy" XP002093654 & ROGOZINA: OFTALMOL ZH, Bd. 29, Nr. 7, 1974, Seiten 540-542,
- DATABASE MEDLINE1974 MARGOLIS: "diabetic retinopathy" XP002093655 & MARGOLIS: "diabetic retinopathy" OFTALMOL ZH, Bd. 29, Nr. 2, 1974, Seiten 150-158,
- DATABASE MEDLINE1969 TANIGUCHI: "experiences with miscellaneous medical treatments of diabetic retinopathy" XP002093656 & TANIGUCHI: NIPPON GANKA KIYO, Bd. 20, Nr. 10, 1969, Seiten 975-976,

## Beschreibung

Die Erfindung betrifft die Prophylaxe und die Therapie der diabetischen Retinopathie und Nephropathie.

Die diabetische Retinopathie und die diabetische Nephropathie sind zwei schwerwiegende Begleit- bzw. Folgeerkrankungen der Diabetes mellitus, also der Zuckerkrankheit. Die Zuckerkrankheit ist eine Störung des Glucosestoffwechsels, die durch einen relativen oder absoluten Mangel an Insulin gekennzeichnet ist.

Das Peptidhormon Insulin wird in den Betazellen des Pankreas gebildet und von dort aus über die Blutbahn im Körper verteilt. Es wirkt dadurch blutzuckersenkend, daß es die Aufnahme der Glucose aus dem Blut in bestimmte Zellen steigert und die Glucose verbrauchenden Stoffwechselvorgänge ankurbelt. Ist die Insulinproduktion erniedrigt, verbleibt daher zuviel Glucose im Blut, und das Krankheitsbild des Diabetes mellitus entwickelt sich.

Der Typ II des Diabetes mellitus entwickelt sich in der Regel aus einem Bündel von Symptomen, welches Bluthochdruck, Übergewicht und Fettstoffwechselstörungen als metabolisches Syndrom umfaßt. Im weiteren Krankheitsverlauf treten Schädigungen des Nervensystems, die sogenannte diabetische Polyneuropathie, des Herzkreislaufsystems sowie die obengenannte diabetische Retinopathie und Nephropathie auf.

Die diabetische Retinopathie (Retinopathia diabetica) ist eine Schädigung der Netzhaut (Retina) des Auges. Dabei tritt eine Mikroangiopathie des Augenhintergrunds, also eine Schädigung der kleinen und kleinsten arteriellen Gefäße des Augenhintergrunds, auf.

Es werden zwei Formen der diabetischen Retinopathie unterschieden. Bei der nicht-proliferativen oder Hintergrundretinopathie treten Netzhautblutungen, Ausweitungen der arteriellen Blutgefäße (sogenanntes Aneurisma), die hier als sogenannte Mikroaneurismen ausgeprägt sind, harte Lipidablagerungen sowie Netzhautödem mit Sehschärfenverlust auf.

Bei der proliferativen Retinopathie treten zusätzlich sogenannte Cotton-Wool-Herde sowie Gefäßneubildungen auf. Vor der Netzhaut kann es zudem zu wiederholten Glaskörperblutungen infolge einer Netzhautischämie, also Durchblutungsstörungen der Netzhaut durch Gefäßverschlüsse kommen. Als Langzeitfolge des Diabetes mellitus, nach ca. 15 Jahren, je nach Stoffwechseleinstellung des Patienten, kann es sogar zu einem neovaskulären Glaukom ("grüner Star") bis zur Erblindung kommen.

Zur Prophylaxe und Therapie der diabetischen Retinopathie sind noch keine Verfahren etabliert, die auf einer Einnahme von Medikamenten beruhen. Eine Behandlung der Krankheit im fortgeschrittenen Stadium, bei dem es zu Netzhautablösungen kommen kann, erfolgt durch Photo- oder Kryokoagulation. Dabei wird die Netzhaut mit den dahinterliegenden Gewebeschichten "verklebt". Eine weitere Art der Behandlung ist die Vitrektomie, d.h. eine mikrochirurgische teilweise oder fast vollständige Entfernung des Glaskörpers.

Diese sehr weitgehenden operativen Behandlungsmethoden sind jedoch nur bei bereits weit fortgeschrittener Schädigung des Augenhintergrunds angebracht und gehen häufig mit Sehverlusten einher.

Die diabetische Nephropathie wird auch diabetische Glomerulosklerose oder Glomerulosklerose Kimmenstiel-Wilson genannt. Bei dieser Nierenschädigung sind die Blutkapillarknäuel, die sogenannten Glomeruli, der Niere geschädigt. Meist treten diese Schädigungen bei mehr als zehn Jahre bestehendem Diabetes mellitus auf. Ca. 20 % aller Diabetiker entwickeln eine Nephropathie.

Die diabetische Nephropathie äußert sich in Proteinurie, also Ausscheidung von Proteinen im Urin, Bluthochdruck und Ödemen, diffuser Verbreiterung der Blutkapillarwände und knötchenartigen Verdickungen unter Einengung des Gefäßvolumens, Ablagerungen in der Kapillarwand sowie Mikroaneurismen.

Die diabetische Nephropathie kann bis zum Nierenversagen führen, in diesem Stadium ist der Patient nur noch durch Dialysebehandlung therapierbar.

Die Ursachen der diabetischen Retinopathie und Nephropathie sind bis heute nicht vollständig aufgeklärt. Als Ursachen werden eine lang andauernde Blutzuckererhöhung, Bluthochdruck, eine verminderte Durchblutung, eine Störung neurotropher Faktoren und oxidativer Streß genannt. Eine der wesentlichsten Ursachen ist die irreversible Glykosilierung von Proteinen, die sogenannte AGE-Entwicklung. Beide Krankheitsbilder sind Begleiterscheinungen der Diabetes, beruhen somit auf derselben Ursache.

Diese identischen Ursachen der diabetischen Retinopathie und Nephropathie ergeben gleiche Ansatzpunkte für die Prophylaxe und Therapie dieser Krankheiten.

So werden beispielsweise blutdrucksenkende Mittel, nämlich ACE-Hemmer und Calciumantagonisten, heute zur Prophylaxe und Therapie der diabetischen Nephropathie verwendet. Diese blutdrucksenkenden Mittel sind jedoch mit klinisch relevanten Nebenwirkungen behaftet, beispielsweise mit Ödemen, gastrointestinalen Störungen, starkem Blutdruckabfall sowie Herzrhythmusstörungen, wodurch sie zur Verwendung in der Prophylaxe ungeeignet sind und ihre Verwendung zur Therapie erschwert wird.

Aus MARGOLIS ET AL "Cocarboxylase in complex treatment of diabetic retinopathy", PROBLEMY ENDOKRINOLOGII, Bd. 5, 1969, Seiten 121 - 122, XP002093652 und DATABASE MEDLINE 1974 MARGOLIS "Diabetic retinopathy" XP002093655 & MARGOLIS "Diabetic retinopathy" OFTALMOL ZH, Bd. 29, Nr. 2, 1974, Seiten 150 - 158, ist bekannt eine Multivitaminbehandlung mit Gehalten an Cocarboxylase im Rahmen einer Kur zur Behandlung der diabetischen Retinopathie durchzuführen. Cocarboxylase ist eine veraltete Bezeichnung des Thiamindiphosphates der physiologisch aktiven Form aller Thiaminderivate. Thiamindiphosphat ist ein wasserlösliches Thiamin.

Es ist Aufgabe der vorliegenden Erfindung, einen Stoff bereitzustellen, der zur Prophylaxe und/oder Therapie der diabetischen Retinopathie und der diabetischen Nephropathie verwendet werden kann.

Erfindungsgemäß wird die Aufgabe durch die Verwendung von lipidlöslichen Thiaminen, insbesondere von Allithiaminen, zur Herstellung eines Arzneimittels zur Prophylaxe und/oder Therapie der diabetischen Retinopathie gelöst.

Die Aufgabe wird ferner durch die Verwendung von lipidlöslichen Thiaminen, insbesondere von Allithiaminen zur Herstellung eines Arzneimittels zur Prophylaxe und/oder Therapie der diabetischen Nephropathie gelöst.

Thiamine sind Vitamine, die auch als Vitamin B₁ bezeichnet werden. Die physiologisch aktive Form aller Thiaminderivate, das Thiaminpyrophosphat (TPP), ist als Koenzym für verschiedene Enzyme des Kohlenhydratstoffwechsels unentbehrlich und kann vom menschlichen Körper selbst nicht hergestellt werden.

Überraschenderweise konnte jetzt gefunden werden, daß lipidlösliche Thiamine zur prophylaktischen und therapeutischen Behandlung der diabetischen Retinopathie sowie der diabetischen Nephropathie eingesetzt werden können. In einem weiter unten näher erläuterten Tierexperiment an "diabetischen Ratten" konnte nämlich festgestellt werden, daß lipidlösliche Thiamine die irreversible Glykosilierung von Proteinen, die AGE-Entwicklung (advanced glycosilation endproducts), inhibieren und damit eine der wesentlichen Ursachen für die Entstehung der diabetischen Retinopathie und Nephropathie unterbinden. Dies war sowohl dann der Fall, wenn das lipidlösliche Thiamin gleichzeitig mit der Entwicklung des Diabetes bei den Tieren eingesetzt wurde (Prophylaxe-Modell) als auch in dem Fall, wenn das lipidlösliche Thiamin erst dann eingesetzt wurde, wenn der Diabetes sich bereits über einen längeren Zeitraum manifestiert hatte (Therapie-Modell).

Lipidlösliche Thiamine sind somit sowohl zur Prophylaxe als auch zur Therapie der diabetischen Retinopathie und Nephropathie geeignet.

Darüber hinaus konnten die Erfinder morphologisch eine Verbesserung der durch Diabetes mellitus bedingten Retina- und Nierenveränderungen beobachten.

Somit wird nun ein Stoff bereitgestellt, der zur Prophylaxe und Therapie der diabetischen Retinopathie und Nephropathie eingesetzt werden kann. Lipidlösliche Thiamine haben den wesentlichen Vorteil, keine Nebenwirkungen aufzuweisen. Als für den Körper lebensnotwendiges Vitamin können Thiamine nämlich in großen Mengen eingenommen werden, ohne daß hierbei Schädigungen des Körpers auftreten.

Ein weiterer Vorteil der lipidlöslichen Thiamine ist es, daß diese oral aufgenommen werden können.

Da lipidlösliche Thiamine eine der wesentlichen Ursachen beider Krankheitsbilder bekämpfen, ist es sowohl zur Monotherapie bzw. -prophylaxe jedes einzelnen Krankheitsbilds als auch zur kombinierten Therapie bzw. Prophylaxe beider Krankheitsbilder geeignet.

Hierbei ist von Vorteil, daß die diabetische Retinopathie und die diabetische Nephropathie häufig gemeinsam auftreten, und mit lipidlöslichen Thiaminen durch einen einzigen leicht einzunehmenden und nebenwirkungslosen Stoff präventiv oder therapeutisch behandelt werden können.

In einem tierexperimentellen Versuch wurde die prophylaktische und therapeutische Wirksamkeit der lipidlöslichen Thiamine über einen Zeitraum von sechs Monaten untersucht. Es eignet sich daher vorzugsweise zur Langzeit-Prophylaxe und/oder Langzeit-Therapie der diabetischen Retinopathie und/oder der diabetischen Nephropathie.

Die Verwendung von Thiaminen zur Langzeit-Therapie hat den Vorteil, daß lipidlösliche Thiamine sehr qut verträglich sind und.darüber hinaus durch die Möglichkeit der oralen Verabreichung besonders leicht einzunehmen sind, somit die sogenannte Patienten-Compliance hervorragend ist.

In einer besonders bevorzugten Ausgestaltung der Erfindung wird als lipidlösliches Thiamin, ein Allithiamin, verwendet.

Lipidlösliche, also fettlösliche Thiamine unterscheiden sich von den wasserlöslichen Thiaminen dadurch, daß der Thiazoliumring des Thiamins in der offenen Thiolform vorliegt und die Thiolgruppe mit einem apolaren Rest modifiziert ist, der entweder aromatisch oder aliphatisch ist. Die Allithiamine gehören zu den lipidlöslichen Thiaminen.

Lipidlösliche Thiamine haben den Vorteil, nach oraler Einnahme hervorragend intestinal resorbiert zu werden (R. Bitsch und I. Bitsch, "Lipidlösliche Thiaminderivate", Deutsche Apothekerzeitung Nr. 2, Seiten 65-68, 1989). Daher werden bei oraler Verabreichung von lipidlöslichen Thiaminen, insbesondere von Allithiaminen, wesentlich höhere Thiaminspiegel im Blut und Gewebe von Organismen erreicht als bei der Gabe gleicher Mengen wasserlöslicher Thiamine. Außerdem werden Allithiamine wesentlich besser im Körper zurückgehalten, d.h. in weniger großen Mengen ausgeschieden als wasserlösliche Thiaminderivate.

In einer höchst bevorzugten Ausgestaltung wird das Allithiamin Benfotiamin, ein aromatisches Thiaminderivat, verwendet.

Bei Benfotiamin handelt es sich um ein Thiamin-pro-Drug, das erst in der Zelle in das physiologisch aktive Thiaminpyrophosphat umgewandelt wird. Benfotiamin hat den pharmakokinetischen Vorzug, dosisproportional resorbiert zu werden. Bei der Verwendung von Benfotiamin ist ferner vorteilhaft, daß nach Benfotiamingabe ein hoher intrazellulärer Thiaminpyrophosphatspiegel erreicht wird, d.h. die relative Bioverfügbarkeit von Benfotiamin ist sehr hoch.

Darüber hinaus weist Benfotiamin eine geringe Toxizität und damit eine hohe Verträglichkeit auf und kann die Bluthirnschranke passieren.

Es versteht sich, daß auch weitere Allithiamine zur Prophylaxe und Therapie von Retinopathien und Nephropathien eingesetzt werden können, beispielsweise die aromatischen Thiaminderivate Bentiamin, Octotiamin oder das nicht-aromatische Fursultiamin. Diese Allithiamine haben zusammen mit Benfotiamin den Vorteil einer hohen Hitzestabilität sowie einer im Vergleich zu den wasserlöslichen Thiaminen erhöhten Resistenz gegenüber Thiaminasen.

Es ist ferner bevorzugt, wenn das lipidlösliche Thiamin in einer Menge von etwa 1 bis 500 mg je Dosis verwendet wird.

In dieser Menge ist eine gute biologische Verwertbarkeit des lipidlöslichen Thiamins durch den Körper möglich.

Als Darreichungsformen des lipidlöslichen Thiamins ist die orale Darreichung besonders vorteilhaft, beispielsweise in Form von Kapseln, Dragees, Tabletten, Filmtabletten, Lutsch-, Kau-, Brausetabletten, Trinklösungen oder Tropfen. Es sind jedoch auch parenterale Darreichungsformen denkbar, beispielsweise in Form von Injektions- oder Infusionslösungen oder als Zäpfchen.

Es versteht sich, daß die vorstehend genannten und die nachstehend noch zu erläuternden Merkmale nicht nur in den angegebenen Kombinationen, sondern auch in Alleinstellung oder in anderen Kombinationen einsetzbar sind, ohne den Rahmen der vorliegenden Erfindung zu verlassen.

Die Erfindung wird nachfolgend im Zusammenhang mit der beiliegenden Zeichnung näher beschrieben und erläutert, in der
- Fig. 1: ein Balkendiagramm zeigt, in dem das Ausmaß der AGE-Entwicklung am Nervus medianus von Ratten mit dem Antikörper 6D12 bestimmt wurde;
- Fig. 2: ein Balkendiagramm entsprechend Fig. 1 zeigt, wobei das Ausmaß der AGE-Entwicklung mit dem Antikörper AG1 bestimmt wurde.
An einem Tiermodell wurde die Auswirkung von Thiaminen auf die irreversible Glykosilierung von Proteinen, die sogenannte AGE-Entwicklung (advanced glycosilation endproducts), untersucht. Die AGE-Entwicklung ist eine der wesentlichen Ursachen der diabetischen Retinopathie und Nephropathie. Sie wurde am Nervus medianus, also dem großen Armnerven der Ratte untersucht. An diesem zum peripheren Nervensystem gehörenden Nerv kann die AGE-Entwicklung aus praktischen Gründen leichter analysiert werden als an den Retinanerven oder den Nierenglomeruli.

Dennoch können die modellhaft an diesen Nerven durchgeführten Untersuchungen auf andere Organe übertragen werden, da die AGEs ubiquitär vorkommen.

Die Untersuchung wurde mit sechs Gruppen mit je 10 bis 13 Ratten durchgeführt. Die Tiere hatten ein Alter von ca. zehn bis zwölf Wochen und ein Gewicht oberhalb 300 g.

Die Gruppe A stellt die Normalkontrolle dar, d.h. die gesunden Tiere blieben unbehandelt. Die Gruppe A umfaßte 13 Tiere.

Bei Gruppe B wurde experimentell eine Diabetes mellitus ausgelöst. Dies erfolgte durch einmalige Vergabe von Streptozotocin in einer Menge von 65 mg/kg Körpergewicht. Streptozotocin schädigt selektiv die Betazellen des Pankreas und unterbindet dadurch die Insulinsekretion. Dadurch wird das Krankheitsbild des Diabetes mellitus bei der Ratte erzeugt. Gruppe B umfaßte 10 Tiere.

Bei Gruppe C wurde direkt nach der Streptozotocin-Vergabe mit einer Benfotiamin-Verabreichung begonnen. Dabei wurden täglich 100 mg/kg Körpergewicht je Dosis an Benfotiamin durch eine Schlundsonde verabreicht. Diese Versuchsgruppe stellt ein Modell für die Prophylaxe gegen die AGE-Entwicklung durch Benfotiamin dar.

In Gruppe D wird direkt nach der Streptozotocin-Vergabe Thiaminnitrat verabreicht, und zwar täglich 70,8 mg/kg je Körpergewicht je Dosis durch eine Schlundsonde. Die verabreichten Mengen an Benfotiamin und Thiaminnitrat waren äquimolar, um die Wirksamkeit von Benfotiamin und Thiaminnitrat vergleichen zu können.

In den Gruppen E und F wurde zunächst die Diabetessituation durch Streptozotocin-Vergabe induziert, es wurde jedoch erst nach zwei Monaten mit der Verabreichung von Benfotiamin (Gruppe E) oder Thiaminnitrat (Gruppe F) begonnen. Die Gruppen E und F sind somit ein Modell für die Verwendung von Thiamin zur therapeutischen Behandlung der diabetischen Retinopathie und der diabetischen Nephropathie.

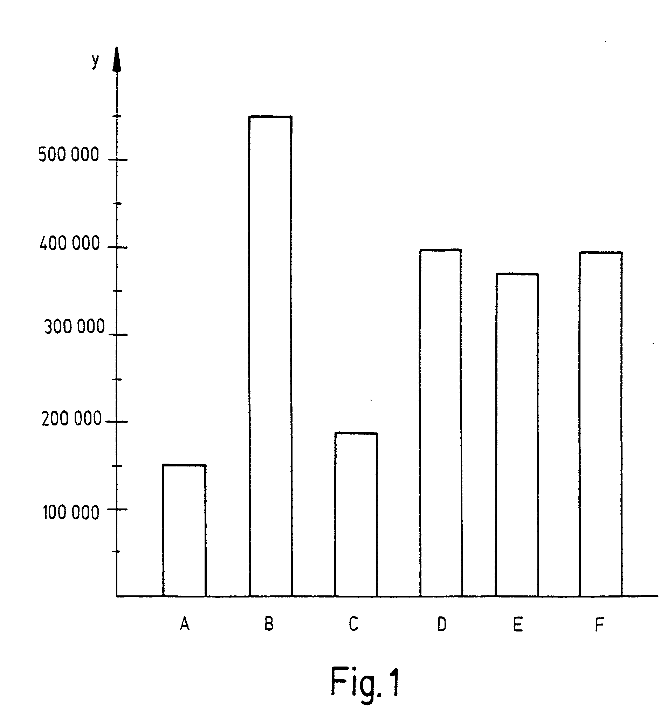

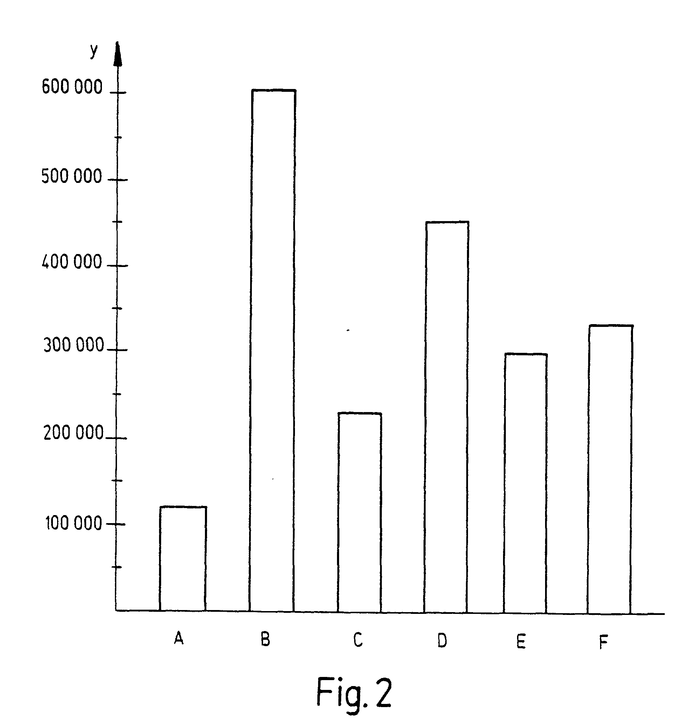

Die Behandlung der Tiere mit dem Thiamin erfolgte für sechs Monate. Dann wurden die Tiere geopfert, und die AGE-Entwicklung am Nervus medianus mit Hilfe zweier Antikörper, dem Antikörper 6D12 (Fig. 1) und dem Antikörper AG1 (Fig. 2) untersucht.

Beide Antikörper sind spezifisch für irreversibel glykosilierte Proteine und können somit direkt zum Nachweis der AGE-Entwicklung eingesetzt werden.

Dazu wurde an Schnitten des Nervus medianus der Tiere aus den sechs oben vorgestellten Gruppen eine sogenannte "Antikörperfärbung" mit dem Fachmann bekannten immunhistochemischen Methoden durchgeführt.

Die Schnitte wurden entweder mit dem Antikörper 6D12 oder dem Antikörper AG1 als Primärantikörper inkubiert. Nach mehrmaligem Waschen zur Entfernung des unspezifisch gebundenen Primärantikörpers wurden die Schnitte mit einem Sekundärantikörper inkubiert, der die Primärantikörper erkennt und mit einem Enzym konjugiert ist, welches eine Farbreaktion katalysieren kann.

Derartige Enzyme sind im Stand der Technik bekannt, meist wird hierzu die Alkalische Phosphatase oder eine Peroxidase eingesetzt.

Nach wiederholtem Waschen zur Entfernung des unspezifisch gebundenen Sekundärantikörpers wird eine Farbreaktion durchgeführt, bei der ein zunächst ungefärbtes Substrat des konjugierten Enzyms durch Enzymkatalyse einen unlöslichen Farbkomplex bildet, der an den Stellen, an denen der Sekundärantikörper und damit auch der Primärantikörper gebunden ist, präzipitiert und im Mikroskop sichtbar ist.

Das Ausmaß an Färbung kann im Mikroskop bestimmt werden und spiegelt direkt das Ausmaß der AGE-Entwicklung wider.

Die Ergebnisse dieses Experiments sind in der nachstehenden Tabelle 1 sowie in den Figuren 1 und 2 wiedergegeben.

Ermittelt wurde die Dichte der Farbpunkte je ausgewertetem Bereich je Milligramm Protein (density/area/mg protein). Die Menge an Protein wurde an einem Proteinextrakt bestimmt. Die entsprechenden Mittelwerte aller Tiere wurden ermittelt, sie sind in den Figuren 1 und 2 mit "Y" bezeichnet.

**TABELLE 1**

| Gruppe | Anzahl der Tiere je Gruppe | Mittelwert± Standardabweichung, 6D12 | Mittelwert± Standardabweichung, AG1 | p-Werte, 6D12 | p-Werte, AG1 |
|---|---|---|---|---|---|
| A Normalkontrolle | 13 | 156 174,48± 91 556,48 | 118 087,97± 129 206,67 | | |
| B Diabetes | 10 | 553 187,94± 221 835,27 | 604 366,24± 194 817,87 | A vs. B: 0,0001 | A vs. B: 0,0001 |
| C Diabetes Benfotiamin Prophylaxe | 11 | 192 969,68± 103 335,51 | 231 508,77± 144 695,82 | C vs. A: 0,3650 C vs. B: 0,0001 | C vs. A: 0,0548 C vs. B: 0,0001 |
| D Diabetes Thiaminnitrat Prophylaxe | 13 | 406 628,78± 141 190,10 | 449 502,72± 214 611,86 | D vs. B: 0,0668 D vs. C: 0,0004 | D vs. B: 0,0889 D vs. C: 0,0092 |
| E Diabetes Benfotiamin Therapie | 12 | 372 023,74± 155 583,78 | 297 713,88± 183 738,05 | E vs. A: 0,0003 E vs. B: 0,0368 E vs. C: 0,004 | E vs. A: 0,0011 E vs. B: 0,0091 E vs. C: 0,3511 |
| F Diabetes Thiaminnitrat Therapie | 13 | 406 543,44± 148 445,24 | 335 482,71± 159 991,88 | F vs. B: 0,0713 F vs. D: 0,9988 | F vs. B: 0,0015 F vs. D: 0,1377 |

In Fig. 1 sind die mit dem Antikörper 6D12 erhaltenen Ergebnisse, in Fig. 2 die mit dem Antikörper AG1 erhaltenen Ergebnisse dargestellt. Die Bestimmung mit zwei verschiedenen Antikörpern, die beide spezifisch AGEs erkennen, dient der Überprüfung der Ergebnisse.

Aus Fig. 1 ist ersichtlich, daß die Menge an nachgewiesenen AGEs bei diabetischen Ratten (Gruppe B), die kein Thiamin erhielten, um das Drei- bis Vierfache höher als bei normalen, gesunden Tieren (Gruppe A) ist. Wird den diabetischen Ratten sofort nach Diabetesinduktion Benfotiamin verabreicht (Gruppe C), so wird die AGE-Entwicklung fast vollständig verhindert, d.h. fast auf das Normalmaß beschränkt. Dies zeigt die hervorragende Wirkung von Benfotiamin.

Auch bei einer Vergabe von wasserlöslichem Thiamin direkt nach Diabetesinduktion (Gruppe D) ergibt sich eine Reduktion der AGE-Entwicklung, die allerdings nicht so ausgeprägt ist wie bei einer Benfotiamin-Verabreichung (Gruppe C) ist.

Bei einer Vergabe von Benfotiamin zwei Monate nach Diabetesinduktion (Gruppe E) ist ebenfalls eine Reduktion der AGE-Entwicklung nachweisbar. Auch die Vergabe von wasserlöslichem Thiamin zwei Monate nach der Diabetesinduktion (Gruppe F) reduziert die AGE-Entwicklung (wenn auch etwas weniger ausgeprägt) im Vergleich zur Diabeteskontrolle ebenfalls.

Dieses Versuchsergebnis wird durch die Untersuchung mit einem weiteren AGE-spezifischen Antikörper, dem Antikörper AG1, bestätigt. Die in Fig. 2 abgebildeten Versuchsergebnisse bestätigen in vollem Umfang das mit dem Antikörper 6D12 erhaltene Ergebnis.

In einer statistischen Auswertung der Versuchsergebnisse wurden die Standardabweichungen sowie die p-Werte ermittelt. Die Ergebnisse sind in Tabelle 1 aufgeführt.

Die p-Werte, auch Irrtumswahrscheinlichkeiten genannt, sind ein Maß dafür, ob sich zwei Werte signifikant voneinander unterscheiden. Je kleiner der p-Wert, desto signifikanter unterscheiden sich zwei Werte voneinander.

Bei einem Vergleich der Meßergebnisse für die AGE-Entwicklung zwischen Gruppe C, nämlich den direkt nach Diabetesinduktion mit Benfotiamin behandelten Ratten, mit der Normalkontrolle, Gruppe A, zeigt sich, daß sich die Werte nicht signifikant unterscheiden. Dagegen unterscheiden sie sich hochsignifikant von den bei den diabetischen Ratten erhaltenen Werte. Das heißt, daß die Benfotiaminbehandlung die AGE-Entwicklung auf das Normalniveau absenkt. Dies belegt die hervorragende Wirksamkeit von Benfotiamin zur Prophylaxe.

Dieses Niveau wird von Thiaminnitrat nicht erreicht, jedoch zeigt sich ein Trend zur Absenkung der AGE-Entwicklung. Unter Berücksichtigung der Tatsache, daß bisher noch keine prophylaktische oder therapeutische Behandlung der diabetischen Retinopathie und Nephropathie bekannt ist, und daß diese Krankheiten sich über lange Zeiträume hinweg ausbilden, ist ein solcher Trend beachtenswert, denn jede Verbesserung der Krankheit hilft dem Patienten.

Wird Benfotiamin erst nach zwei Monaten der Diabetes-Entwicklung verabreicht, so zeigt sich, daß hier ein signifikanter Unterschied zur unbehandelten Diabetessituation nachweisbar ist. Dies belegt die hervorragende Wirksamkeit von Benfotiamin zur Therapie. Das gleiche gilt auch für die Behandlung mit wasserlöslichem Thiamin, wobei hier der Effekt wieder weniger ausgeprägt ist.

Zusammenfassend ergibt sich, daß eine Verabreichung von Benfotiamin direkt bei Beginn der Krankheit Diabetes mellitus die AGE-Entwicklung fast auf das im gesunden Körper vorhandene Ausmaß beschränkt.

Somit wird durch Vergabe eines lipidlöslichen Thiamins, insbesondere von Benfotiamin, eine der Ursachen der diabetischen Retinopathie und Nephropathie unterbunden und somit die Entwicklung dieser erst im späteren Verlauf des Diabetes mellitus auftretenden Krankheitsbilder präventiv verhindert. Dies bedeutet, daß Benfotiamin zur Prophylaxe der diabetischen Retinopathie und Nephropathie wirksam eingesetzt werden kann.

Die Ergebnisse zeigen auch, daß Benfotiamin wirksam bei der Therapie von diabetischer Retinopathie und Nephropathie eingesetzt werden kann. Werden diese Wirkstoffe erst dann verabreicht, wenn sich die Krankheit Diabetes mellitus bereits einige Zeit manifestiert hat und ein für die Diabetes typisches hohes Ausmaß an AGEs sich bereits entwickelt hat, so kann durch Verabreichung des Wirkstoffes diese Entwicklung reduziert werden. Eine der Ursachen der beiden Diabetesfolgekrankheiten wird somit durch Benfotiamin wirksam therapeutisch bekämpft.

Die beiden eingesetzten Thiamine, nämlich Benfotiamin und Thiaminnitrat, sind bezüglich der Bioverfügbarkeit als Extreme anzusehen. Benfotiamin ist eines der besten bioverfügbaren Thiaminderivate, Thiaminnitrat eines der am schlechtesten bioverfügbaren Thiaminderivate.

## Patentansprüche

1. Verwendung von lipidlöslichen Thiaminen, insbesondere von Allithiaminen zur Herstellung eines Arzneimittels zur Prophylaxe und/oder Therapie der diabetischen Retinopathie.

2. Verwendung von lipidlöslichen Thiaminen, insbesondere von Allithiaminen zur Herstellung eines Arzneimittels zur Prophylaxe und/oder Therapie der diabetischen Nephropathie.

3. Verwendung nach Anspruch 1 oder 2 für Langzeit-Prophylaxe und/oder Langzeit-Therapie.

4. Verwendung nach einem der Ansprüche 1 bis 3, **dadurch gekennzeichnet, dass** als lipidlösliches Thiamin Benfotiamin verwendet wird.

5. Verwendung nach einem der Ansprüche 1 bis 4, **dadurch gekennzeichnet, dass** das Thiamin in einer Menge von etwa 1 bis 500 mg je Dosis verwendet wird.

## Claims

1. Use of lipid-soluble thiamines, in particular of allithiamines, for producing a medicament for the prophylaxis and/or therapy of diabetic retinopathy.

2. Use of lipid-soluble thiamines, in particular of allithiamines, for producing a medicament for the prophylaxis and/or therapy of diabetic nephropathy.

3. Use according to Claim 1 or 2 for long-term prophylaxis and/or long-term therapy.

4. Use according to any of Claims 1 to 3,
**characterized in that** benfotiamine is used as lipid-soluble thiamine.

5. Use according to any of Claims 1 to 4,
**characterized in that** the thiamine is used in an amount of about 1 to 500 mg per dose.

## Revendications

1. Utilisation de thiamines liposolubles, en particulier d'allithiamines pour la production d'un médicament destiné à la prophylaxie et/ou à la thérapie de la rétinopathie diabétique.

2. Utilisation de thiamines liposolubles, en particulier d'allithiamines pour la production d'un médicament destiné à la prophylaxie et/ou à la thérapie de la néphropathie diabétique.

3. Utilisation selon la revendication 1 ou 2 pour la prophylaxie à long terme et/ou la thérapie à long terme.

4. Utilisation selon l'une quelconque des revendications 1 à 3, **caractérisée en ce que** l'on utilise la benfothiamine en tant que thiamine liposoluble.

5. Utilisation selon l'une quelconque des revendications 1 à 4, **caractérisée en ce que** la thiamine est utilisée en une quantité d'environ 1 à 500 mg par dose.
